# EUROPEAN PATENT SPECIFICATION

(11) **EP 2 087 924 B1**
(45) Date of publication and mention of the grant of the patent: **24.01.2018**
(21) Application number: 08002307.0
(22) Date of filing: 07.02.2008
(51) Int. Cl.: A61N 5/10

(54) **Method for generating and visualizing an ion beam profile, treatment planning system and computer software**
Verfahren zur Erzeugung und Sichtbarmachung eines Ionenstrahlprofils, Behandlungsplanungssystem und Computersoftware
Procédé de génération et de visualisation d'un profil de faisceau d'ions, système de programmation de traitement et logiciel informatique

(43) Date of publication of application: 12.08.2009
(73) Proprietor: Varian Medical Systems Particle Therapy GmbH, 53842 Troisdorf (DE)
(72) Inventor: Murali, Shettihalli, 560026 Bangalore (IN)
(74) Representative: Sedlacek, Anton

(56) References cited:
- WO-A-91/14397
- PURDY J A ET AL: "Advances in 3-dimensional radiation treatment planning systems: room-view display with real time interactivity." INTERNATIONAL JOURNAL OF RADIATION ONCOLOGY, BIOLOGY, PHYSICS 15 NOV 1993, vol. 27, no. 4, 15 November 1993 (1993-11-15), pages 933-944, XP008095553 ISSN: 0360-3016
- PELIZZARI C A ET AL: "Volume visualization in radiation treatment planning." CRITICAL REVIEWS IN DIAGNOSTIC IMAGING DEC 2000, vol. 41, no. 6, December 2000 (2000-12), pages 379-401, XP008095549 ISSN: 1040-8371
- REYNOLDS R A ET AL: "An algorithm for three-dimensional visualization of radiation therapy beams." MEDICAL PHYSICS 1988 JAN-FEB, vol. 15, no. 1, January 1988 (1988-01), pages 24-28, XP007905469 ISSN: 0094-2405
- BEAUDRÉ A ET AL: "[Virtual simulation: means and methodology]" CANCER RADIOTHÉRAPIE : JOURNAL DE LA SOCIÉTÉ FRANÇAISE DE RADIOTHÉRAPIE ONCOLOGIQUE 1997, vol. 1, no. 5, 1997, pages 573-580, XP007905470 ISSN: 1278-3218
- KRAFT, G: "Tumortherapy with ion beams", NUCLEAR INSTRUMENTS & METHODS IN PHYSICS RESEARCH. SECTION A: ACCELERATORS, SPECTROMETERS, DETECTORS, AND ASSOCIATED EQUIPMENT, ELSEVIER BV * NORTH-HOLLAND, NL, vol. 454, no. 1, 1 November 2000 (2000-11-01), pages 1-10, XP004238218, ISSN: 0168-9002, DOI: 10.1016/S0168-9002(00)00802-0

## Description

The present invention relates to a method for generating and visualizing a profile of an ion beam. The present invention also relates to a computer software product and a treatment planning system for generating and visualizing an ion beam profile. In particular, ion beam profiles are used during treatment planning for a particle therapy treatment.
Particle therapy is commonly used in the field of oncology to treat localized forms of cancer as well as other afflictions. For particle therapy, particles like protons, carbon ions or other particles are accelerated to high energies and formed to a beam which is directed to a target volume. The particles of the particle beam interact with the target in a relatively delimited region resulting in localized cell damage with minimal damage of the surrounding healthy tissue. The technique of particle therapy however can also be used in fields, where no treatment of a patient occurs, for example in research and development activities which involve irradiation of materials or phantoms.
Particle therapy requires accurate treatment planning. Usually, treatment planning systems are computer-based applications used for generating treatment plans based on patient images, target definition and machine geometry definition. Typically, treatment planning systems allow the clinical user to visualize the three-dimensional volume of patient images, beam shapes, automatic beam shaping, placement and dose calculation.
After dose calculation, a graphical representation of the dose distribution that would affect the target volume and the surrounding tissue can be visualized. Such a dose distribution allows a user to verify whether the ion beam would affect the tissue in a desired way. Since dose calculation is based on the physical characteristics of an ion beam, a visualization of ion beam properties created with dose calculation algorithm also reflects the physical characteristics of an ion beam. Dose calculation, however, is rather time-consuming and can slow down the process of treatment planning. Especially, when a user changes parameters - as e.g. the incidence direction of the ion beam - several times during treatment planning, doses have to be recalculated several times.
Purdy J et al: "Advances in 3-dimensional radiation treatment planning systems: room-view display with real time interactivity", International Journal of Radiation Oncology, Biology, Physics, 15 Nov 2003, vol. 27(4): 933-944, describes a radiation treatment planning system that displays virtual radiation beams by specifying an incidence direction and a target region. This document is considered the closest prior art with regard to the method of claim 1. Pelizzari CA et al, "Volume visualization in radiation treatment planning", Crit Rev Diagn Imaging. 2000 Dec; 41(6): 379-401, discloses the use of wireframe or shaded surface models of proposed radiation beams displayed on a display.
Reynolds RA et al, "An algorithm for three-dimensional visualization of radiation therapy beams", Med Phys. 1988 Jan-Feb; 15(1): 24-28, disclose a computer algorithm to display radiation beam superimposed on 3-D views of patient anatomy.
Beaudre A et al, "Simulation virtuelle: moyens et methodologie », Cancer RadioThérapie 1997, 1(5): 573-580, describes a virtual simulation of the irradiation technique that allows definition in 3D of the optimized geometrical characteristics of the treatment beams with respect to anatomical structures of the patient.
WO 91/14397 discloses a method for combining real or actual 3-D scanning or imaging data with simulated graphics so that the 3-D nature of structures within a given area or region can be viewed in relationship to the original image data.
It is an object of the present invention to provide a method for generating and visualizing a profile of an ion beam which is fast, reliable and easy to implement and which respects the particular characteristics of an ion beam. Further, it is an object of the present invention to provide a therapy planning system and a computer software which allows a fast and reliable determination and visualization of an ion beam profile.
The object of the invention is achieved by a method according to claim 1, by a treatment planning system according to claim 5 and by a computer software according to claim 6. The method relies substantially only on the geometrical properties of the target region and on the incidence direction of the ion beam. It can be applied to a two-dimensional target region (leading to a two-dimensional ion beam profile) as well as to a three-dimensional target region (leading to a three-dimensional ion beam profile). For example, the target region can be a two or three-dimensional representation of the volume that is to be irradiated by the ion beam.
Compared to methods that visualize characteristics of an ion beam by presenting a dose distribution based on a dose calculation, this method provides a fast generation and visualization of the ion beam profile. The method does not require any dose calculation in order to create and/or to visualize an ion beam profile.
Before carrying out the method steps, a two or three dimensional image data set comprising an image of the target region is provided. Such an image data set can be created for example by using three-dimensional medical imaging methods as computer tomography or magnetic resonance imaging. The incidence direction of a particle beam and the target region are specified using the image data set. A user designates the incidence direction and the target region on images created from the image data set. For example, the target region can be generated from a set of contours delineated in images of the image data set. This would allow e.g. to create a mesh representing the target region.
By applying this method, it is assured that the ion beam profile created reflects the typical characteristics of an ion beam. The ion beam profile is orthographic, has a well-defined penetration depth and it is correctly associated with the target region. The method can thus be advantageously used in planning software or in visualization software for oncology particle therapy systems. The method allows a user to quickly create an ion beam profile for easy verification of the shape of the ion beam profile.

Furthermore, the method can even be used in connection with the pencil beam scanning technique. In this case, the ion beam profile indicates how the situation would be if all the pencil beams would be switched on at the same time. The ion beam profile thus indicates the complete region that gets exposed to the ion beam.

The incidence direction can for example be directly specified on a graphical display. For example, a user can use an input device like a mouse and specify the incidence direction. The incidence direction can be specified indirectly as well, e.g. by specifying the position of the beam source.

When displaying the ion beam profile, it is preferably displayed together with images created from the image data set with corresponding spatial relationship. The ion beam profile can for example be displayed together with structures of the object which is to be irradiated, for example together with the target region, together with the target region and the surrounding structures etc. This allows a user to easily verify whether the ion beam profile passes the object at the right location or not.

The incidence direction of the particle beam and/or the target region can be varied several times and the ion beam profile can be re-created and re-displayed after each variation.

Once it has been determined that the ion beam profile fits the needs of the treatment, a dose calculation can be performed based on the geometrical properties of the target region and of the incidence direction of the particle beam in a subsequent step. Dose calculation however is performed only as a last final step. After dose calculation, a graphical representation of a dose distribution can be visualized so as to precisely show how the target region at the surrounding structures would be affected by the ion beam.

The target region can comprise one single connected target volume. The method can be however also applied to a complex target region which comprises two or more disconnected target volumes.

In more detail, the step of creating the ion beam profile comprises the following steps:
moving a virtual plane orthogonal to said incidence direction through the target region, thereby collecting contours of intersection of said virtual plane with said target region and
creating said ion beam profile using said contours.

The contours of intersection of the virtual plane with the target region are used to create the ion beam profile that reflects the physical properties of the ion beam. This method can easily be implemented e.g. on a computer system.

In more detail, the step of creating the ion beam profile comprises the following steps:
(a) generating a virtual plane orthogonal to said incidence direction
(b) positioning said virtual plane through said target region at a distal edge of said target region
(c) determining an intersection of said virtual plane with said target region
(d) expanding said ion beam profile by the contour of said intersection
(e) moving said virtual plane against the incidence direction of said particle beam
(f) repeating step (c) to (e) until said virtual plane has completely moved through said target region.

When the virtual plane moves through the target region thus covering the target region, the ion beam profile stepwise expands and therefore the profile constantly increases when moving from the downstream side of the target region to the upstream side of the target region.

When the ion beam profile is generated by moving the virtual plane through the target region, the contours of the intersections of the virtual planes with the target region can for example be used to create a mesh characterizing the ion beam profile. By repeating step (c) to (e) the mesh gradually builds up, with an increasing contour when moving from the distal edge of the target region to the proximal edge of the target region.

The ion beam profile characterized by a mesh can easily be visualized on a graphical display. Hereby, well-known algorithms for representing a mesh like structure on a graphical display can be used.

With this algorithm, the method can easily be implemented into treatment planning systems. The points contributing to the ion beam profile fulfill the requirements that they are located either on a downstream side of the target region or that they project onto the contour of the target region with respect to said incidence direction.

The treatment planning system comprises at least one input device, at least one output device and a computing unit, wherein the treatment planning system is adapted to perform a method as claimed in one of claims 1 to 4.

The computer software implements a method as claimed in one of the claims 1 to 4 on a computer if the computer software runs on the computer. It can be stored e.g. on a computer-readable medium.

Aspects of the present invention and modifications according to limitations of the dependent claims are shown in the figures without being limited hereon.
FIG. 1 shows an overview of a treatment planning system,
FIG. 2 shows a schematic diagram of a method for generating and visualizing an ion beam profile,
FIG. 3 to FIG. 7 illustrate method steps of the method for generating and visualizing an ion beam profile in more detail,
FIG. 8 to FIG. 10 show some examples of generated ion beam profiles according to the described method for different constellations of target regions.

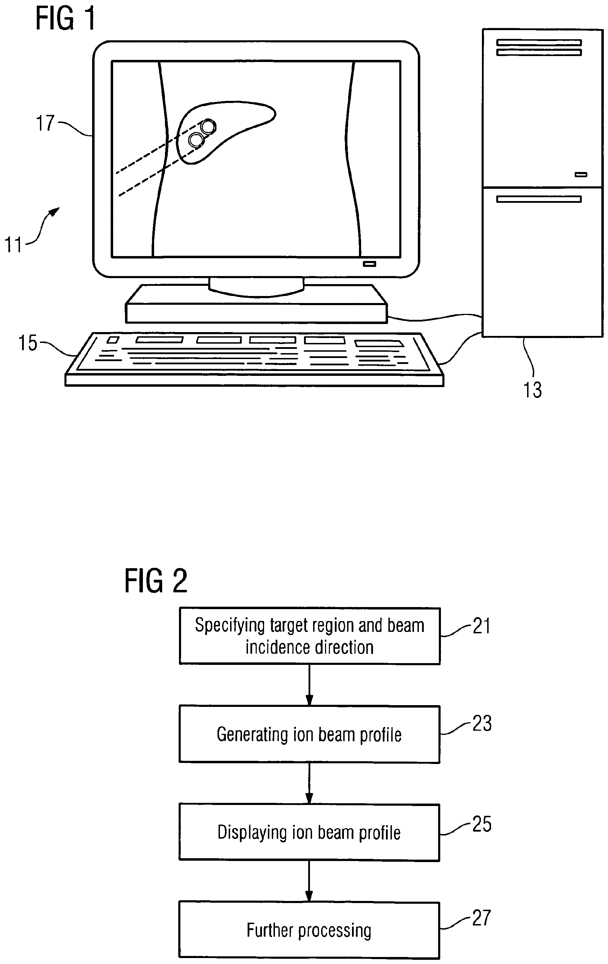

FIG. 1 shows a treatment planning system 11. The treatment planning system comprises a computing device 13 for performing calculations necessary during treatment planning. It comprises further at least one input device 15 that allows a user to interact with the treatment planning system 11 and at least one output device 17 for presenting all necessary information. Usually, a treatment planning system 11 is a computer, on which appropriate treatment planning software runs. FIG. 2 shows a schematic diagram of a method for generating and visualizing an ion beam profile. First (step 21), a target region and an incidence direction of the particle beam are specified. The target region and the incidence direction of the particle beam are specified by using images created from an image data set of an object which is to be irradiated. A user can e.g. delineate sketches of the target region on an image of the image data set by using the input device.
Next (step 23), the ion beam profile is generated only by using geometrical properties of the target region and its spatial relationship to the incidence direction of the particle beam. The ion beam profile is created from points that are located on a downstream side of the target region and from points that are located in front of the target region and project onto the contour of the target region with respect to the incidence direction.

After the ion beam profile has been generated, a graphical representation of the ion beam profile is displayed (step 25).

Since on the downstream side of the target region the ion beam profile is created from points which lie on the outline of the target region and since on the upstream side of the target region the ion beam profile is created from points which project onto the contour of the target region - with respect to the incidence direction -, the ion beam profile reflects the physical properties of an ion beam. Only based on geometrical properties of the target region and its spatial relationship to the incidence direction of the particle beam, the ion beam profile allows nonetheless visualizing the correct shape of the ion beam profile. In particular, if the ion beam profile is visualized together with structures of the object which is to be irradiated, a user can easily verify if the ion beam profile intersects with important structures or not.

If desired, a further optional step can be carried out (step 27). This step can comprise:
- Varying said incidence direction of said particle beam and/or said target region
- Re-creating and re-displaying said ion beam after each variation
- Accepting one of said varied incidence direction and/or one of said varied target region as a final incidence direction and/or as a final target direction, respectively,
- Calculating a dose distribution for the ion beam based on the final incidence direction and/or on the final target region.

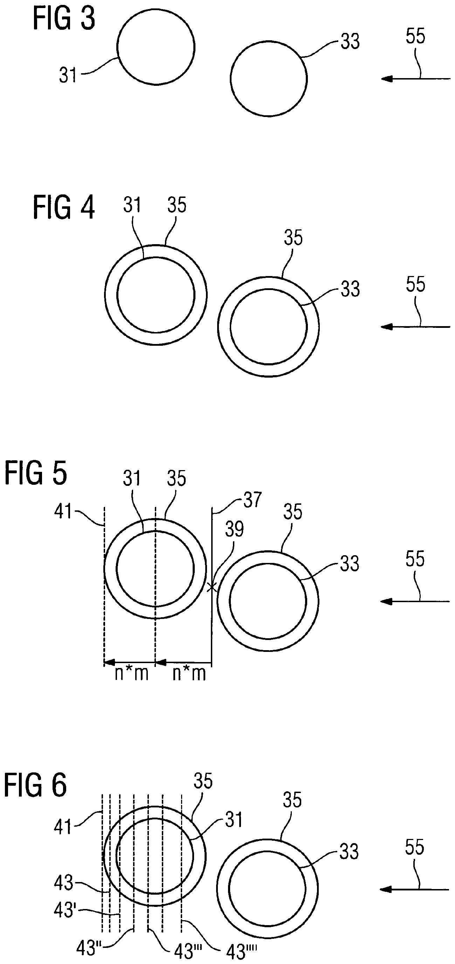

FIG. 3 to FIG. 6 illustrate method steps of a method for generating and visualizing an ion beam profile in more detail.

As a first step, the target region and the beam characteristics as the source position and/or the incidence direction of the beam are specified. A predefined safety margin or a safety margin derivable from beam characteristics can be specified as well. The target region can comprise several separated target volumes. It is assumed that the target volumes are of nonzero volume. The target volumes can be listed in a target list.

In the first step, all the parameters necessary for the subsequent method steps are specified. This can be done interactively by a user via a graphical user interface, for example.

FIG. 3 shows an example of a target region comprising two separated target volumes, a first target volume 31 and the second target volume 33, each of nonzero volume. The incidence direction 55 is indicated by an arrow.

As the second step, a three-dimensional margin is applied to the target region. Each of the target volumes in the target list is increased by an amount specified in the safety margin. The safety margin can for example be predefined or depend on specified beam characteristics. The second step gives out new targets after applying the margin on all the three directions. This step is optional. However, if desired, it is also possible to generate an ion beam profile only based on the original target volumes.

FIG. 4 shows the target volumes of FIG. 3 which have been expanded by a (three-dimensional) margin 35.

Input for the second step: List of targets, e.g. first target volume 31 and second target volume 33 as shown in Fig. 4. Output of the second step: Expanded target volumes after applying a (3-D) margin.

As a third step, a starting plane is identified. For collecting contours, a starting plane which should be ideally the last plane along the direction of the beam which cuts the target is to be identified. The ideal start position for identifying this plane can be the beam isocenter. At the isocenter position, a plane is created that is perpendicular to the beam direction. The ideal starting plane can then be identified by applying the following steps:
- Cut the target/targets from this plane to check whether it passes through any of the targets
- If it cuts the target/targets move the plane farther away from the beam source by n times the margin m (n is used for faster navigation purpose and is a natural number)
- If it does not cut the target/targets then move the plane towards the beam source (after confirming that there are no further targets).
- Repeat this until the last region in which the edge lies is identified.
- Identify the last plane by dividing the region into two halves and by checking in which region the edge lies.

FIG. 5 shows the expanded target volumes as shown in FIG. 4 together with a plane 37 which is orthogonal to the beam direction and passes through the beam isocenter 39. Starting from this plane 37, the ideal starting plane 41 is identified by using the algorithm described above.

Input for the third step: Expanded target region and a plane 37 orthogonal to the beam incidence direction positioned at the beam isocenter 39 as shown in FIG. 5.
Output of the third step: starting plane 41 as shown in FIG. 5.

As a fourth step, the contour of the ion beam profile is collected by moving the plane. The contour of the ion beam profile can be collected stepwise by using the following algorithm:
- Cut targets from the identified starting plane; unify all the contours obtained. Collect the resulting contours in a separate list output contours list.
- Move the plane towards the source by distance equal to the predefined margin or slightly less than margin. Since margin is usually very small no variations in target shape will be missed. Alternatively, the plane can be moved by an amount of several times of the margin. Alternatively, the plane can be moved by a distance entered by a user that is small compared to the target/targets. However, care should be taken that the amount the plane is shifted by is small enough in order to get an ion beam profile enclosing the targets completely. If the amount the plane is shifted by is too large, the ion beam profile could eventually not cover the targets completely.
- Cut the targets by passing new plane collect resultant contours, project the contours of previous plane onto this plane and unify the resultant contours with the projected contours.
- Repeat this process till all the targets are covered.
- Now create a plane at the beam exit point and project the contours of last plane (obtained after covering all the targets) and collect in the output contours list.

FIG. 6 shows gradually increasing contours as they are collected when the starting plane 41 is shifted stepwise through the target region (shifted planes 43 ... 43'''').

Input for the fourth step: starting plane 41 and a predefined margin 35 as shown in FIG. 6
Output of the fourth step: list of contours obtained by cutting the targets with shifted planes 43 ... 43''''.

As a fifth step, a mesh is generated from the list of contours. All the identified contours collected in an output contours list are passed. A mesh is generated using a mesh generation algorithm. The mesh can be visualized by an ion beam 3D visualization.

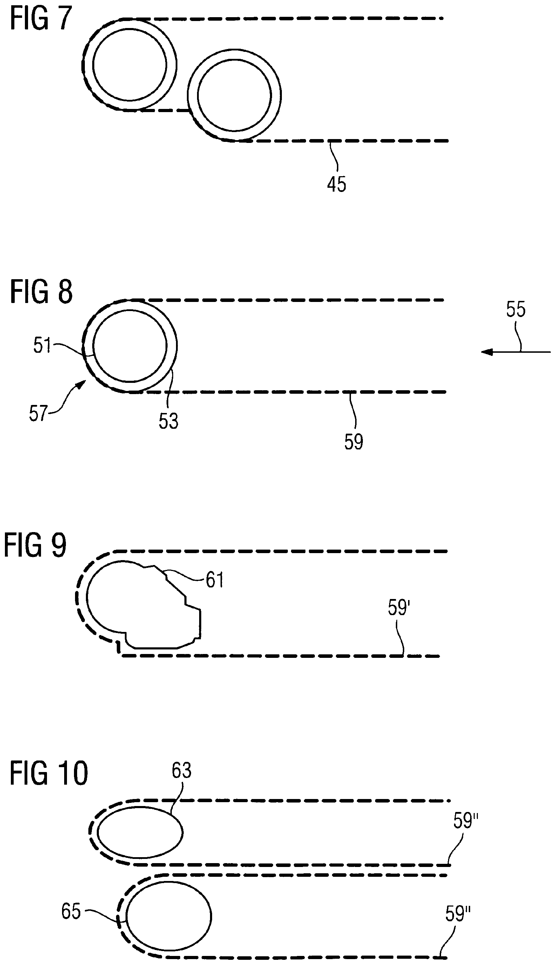

FIG. 7 shows the completed ion beam profile 45 as created by the method.

Input of the fifth step: list of contours.
Output from the fifth step: list of facets (triangles) which define a 3D ion beam profile 45 as shown in Fig. 7

Several sub-algorithms used in the method can be implemented separately. This allows a modular design of the method. Such sub-algorithms are for example:
Union Algorithm: This algorithm takes the list of contours on the same plane and gives out list of contours after applying union on input contours.
Plane Intersection Algorithm: This algorithm takes a plane and a target list and gives out list of contours as a result of intersection of plane with targets.
3D Margin Algorithm: This algorithm takes the list of targets and a predefined margin and gives out new targets after applying the margin on all the three directions.
Mesh Generation Algorithm: This algorithm takes a list of contours and generates a list of facets (triangles) by connecting the contours.

After having applied the method steps as described, the method outputs a list of facets which gives the three-dimensional ion beam profile.

The list of facets can be used for visualizing the three-dimensional ion beam profile by using well-known visualization algorithms. The ion beam profile is preferably visualized together with images of the object which is to be irradiated such that a user can directly see which structures of the object are affected by the ion beam.

FIG. 8 to FIG. 10 shows an ion beam profile, respectively, as created by the method for different shapes of a target region other than the shape of the target region as shown in FIG. 3 to FIG. 7

FIG. 8 shows another ion beam profile 59 as generated by the method for a simple single target volume 51 that has been expanded to a target region 53 by applying a safety margin. The incidence direction 55 of the particle beam defines a downstream side 57 of the target region 53. The ion beam profile 59 comprises points that are located on this downstream side of the target region 53. Moreover, the ion beam profile 59 comprises also points that are located on the upstream side of the target region 53 if these points project with respect to the incidence direction 55 onto the contour of the target region 53.

FIG. 9 shows another ion beam profile 59' as generated by the method for a single irregular-shaped target volume 61. In this case the target is of irregular shape such that the outer edge is broader than the inner edge. The corresponding ion beam profile 59' respects the irregular shape of the target region.

FIG. 10 shows another ion beam profile 59" for a target region which comprises two separate target volumes 63, 65. In FIG. 10 the target volumes 63, 65 do not overlap when viewed from the direction of the particle beam. In this case, the ion beam profile 59" generated by the method corresponds to multiple ion beam profiles covering the clearly separated the target volumes.

## Claims

1. Method for generating and visualizing a profile of an ion beam, the method comprising following steps:
- Specifying an incidence direction (55) of a particle beam on images created from an image data set of an object which is to be irradiated,
- Specifying a target region (53) that is to be irradiated by said particle beam on the images created from an image data set of an object which is to be irradiated,
- Creating an ion beam profile (45, 59, 59', 59") from points that lie on an outline of said target region (53) and that are located on a downstream side (57) of said target region (53) and
from points that are located in front of said target region (53) and project onto the contour of said target region (53) with respect to said incidence direction (55),
- Displaying a graphical representation of said ion beam profile (45, 59, 59', 59"),
wherein the step of creating an ion beam profile (45, 59, 59', 59") comprises:
moving a virtual plane orthogonal to said incidence direction through said target region, thereby collecting contours of intersection of said virtual plane with said target region and
creating said ion beam profile using said contours.

2. Method as claimed in claim 1, wherein
the step of specifying a target region comprises:
- Selecting one or more target volumes (31, 33, 51, 61, 63, 65),
- Expanding said one or more target volumes (31, 33, 51, 61, 63, 65) by a safety margin (35),
- Determining as said target region (53) the union of said one or more expanded target volumes.

3. Method as claimed in claim 1 or 2, wherein
the step of creating an ion beam profile (45, 59, 59', 59") comprises:
(a) Generating a virtual plane orthogonal (37) to said incidence direction (55)
(b) Positioning said virtual plane through said target region at a distal edge of said target region (53)
(c) Determining an intersection of said virtual plane with said target region (53)
(d) Expanding said ion beam profile by said intersection
(e) Moving said virtual plane upstream to said incidence direction (55)
(f) Repeating step (c) to step (e) at least until said virtual plane has moved through said target region.

4. Method as claimed in one of claims 1 to 3, further comprising the steps:
- Varying said incidence direction of said particle beam and/or said target region
- Re-creating and re-displaying said ion beam profile after each variation
- Accepting one of said varied incidence direction and/or one of said varied target region as a final incidence direction and/or as a final target region, respectively,
- Calculating a dose for the ion beam based on said final incidence direction and/or on said final target region.

5. Treatment planning system comprising
at least one input device (15), at least one output device (17) and a computing unit (13), wherein said treatment planning system (11) is adapted to perform a method as claimed in one of claims 1 to 4.

6. A computer-readable medium having computer executable instructions for performing a method recited in any one of claims 1 to 4.

## Patentansprüche

1. Verfahren zur Erzeugung und Sichtbarmachung eines Ionenstrahlprofils, wobei das Verfahren die folgenden Schritte umfasst:
- Festlegen einer Einfallsrichtung (55) eines Teilchenstrahls auf aus einem Bilddatensatz eines Objekts gebildeten Bildern, das zu bestrahlen ist,
- Festlegen eines Zielbereichs (53), der von dem Teilchenstrahl zu bestrahlen ist, auf den aus einem Bilddatensatz eines zu bestrahlenden Objekts ist, gebildeten Bildern,
- Bilden eines Ionenstrahlprofils (45, 59, 59', 59") aus Punkten, die auf einem Umriss des besagten Zielbereichs (53) liegen und die sich auf einer stromabwärtigen Seite (57) des besagten Zielbereichs (53) befinden, und
aus Punkten, die vor dem Zielbereich (53) positioniert sind bezüglich der Einfallsrichtung (55) auf die Kontur des Zielbereichs (53) vorragen,
- Anzeigen einer graphischen Darstellung des besagten Ionenstrahlprofils (45, 59, 59', 59"),
wobei der Schritt des Bildens eines Ionenstrahlprofils (45, 59, 59', 59") umfasst:
Bewegen einer virtuellen Ebene orthogonal zu der Einfallsrichtung durch den Zielbereich, wodurch Schnittkonturen besagter virtueller Ebene mit besagtem Zielbereich gesammelt werden, und
Bilden des besagten Ionenstrahlprofils unter Verwendung dieser Konturen.

2. Verfahren nach Anspruch 1, wobei der Schritt des Festlegens eines Zielbereichs Folgendes umfasst:
- Auswählen eines Zielvolumens oder mehrerer Zielvolumina (31, 33, 51, 61, 63, 65),
- Erweitern des einen Zielvolumens oder der mehreren Zielvolumina (31, 33, 51, 61, 63, 65) um eine Sicherheitsmarge (35),
- Festlegen der Vereinigung des einen erweiterten Zielvolumens oder der mehreren erweiterten Zielvolumina als Zielbereich (53).

3. Verfahren nach Anspruch 1 oder 2, wobei der Schritt des Bildens eines Ionenstrahlprofils (45, 59, 59', 59") Folgendes umfasst:
(a) Erzeugen einer virtuellen Ebene orthogonal (37) zu besagter Einfallsrichtung (55),
(b) Positionieren der virtuellen Ebene durch besagten Zielbereich an einer distalen Kante des besagten Zielbereichs {53),
(c) Bestimmen einer Überschneidung besagter virtueller Ebene mit dem Zielbereich (53),
(d) Erweitern des besagten Ionenstrahlprofils um besagte Überschneidung,
(e) Bewegen besagter virtueller Ebene stromaufwärts zu besagter Einfallsrichtung (55),
(f) Wiederholen von Schritt (c) bis Schritt (e), zumindest bis sich die virtuelle Ebene durch besagten Zielbereich bewegt hat.

4. Verfahren nach einem der Ansprüche 1 bis 3, ferner umfassend die folgenden Schritte:
- Variieren der Einfallsrichtung des besagten Teilchenstrahls und/oder des besagten Zielbereichs,
- nochmaliges Bilden und nochmaliges Anzeigen des besagten Ionenstrahlprofils nach jeder Variation,
- Annehmen der variierten Einfallsrichtung und/oder des variierten Zielbereichs als endgültige Einfallsrichtung und/oder als endgültigen Zielbereich,
- Berechnen einer Dosis für den Ionenstrahl auf Basis der besagten endgültigen Einfallsrichtung und/oder des endgültigen Zielbereichs.

5. Behandlungsplanungssystem, umfassend zumindest eine Eingabevorrichtung (15), zumindest eine Ausgabevorrichtung (17) und eine Recheneinheit (13), wobei besagtes Behandlungsplanungssystem (11) dazu eingerichtet ist, ein Verfahren nach einem der Ansprüche 1 bis 4 durchzuführen.

6. Computerlesbares Medium mit computerausführbaren Anweisungen zum Durchführen eines Verfahrens nach einem der Ansprüche 1 bis 4.

## Revendications

1. Procédé de génération et de visualisation d'un profil d'un faisceau d'ions, le procédé comprenant les étapes suivantes :
- spécifier une direction d'incidence (55) d'un faisceau de particules sur des images créées à partir d'un jeu de données d'image d'un objet devant être irradié ;
- spécifier une région cible (53) à irradier par ledit faisceau de particules sur les images créées à partir d'un jeu de données d'image d'un objet devant être irradié ;
- créer un profil de faisceau d'ions (45, 59, 59', 59") à partir de points qui se trouvent sur un tracé de ladite région cible (53) et se situent sur un côté aval (57) de ladite région cible (53), et à partir de points qui se situent devant ladite région cible (53) et se projettent sur le contour de ladite région cible (53) par rapport à ladite région d'incidence (55) ;
- afficher une représentation graphique dudit profil de faisceau d'ions (45, 59, 59', 59" ),
dans lequel l'étape de création d'un profil de faisceau d'ions (45, 59, 59', 59") comprend les étapes pour :
déplacer un plan virtuel de manière orthogonale par rapport à ladite direction d'incidence à travers ladite région cible, collectant ainsi des contours d'intersection entre ledit plan virtuel et ladite région cible, et
créer ledit profil de faisceau d'ions à l'aide desdits contours.

2. Procédé selon la revendication 1, dans lequel l'étape pour spécifier une région cible comprend les étapes suivantes :
- sélectionner un ou plusieurs volumes cibles (31, 33, 51, 61, 63, 65) ;
- agrandir ledit ou lesdits volumes cibles (31, 33, 51, 61, 63, 65) à raison d'une marge de sécurité (35) ;
- déterminer comme ladite région cible (53) l'union dudit ou desdits volumes cibles agrandis.

3. Procédé selon la revendication 1 ou 2, dans lequel l'étape pour créer un profil de faisceau d'ions (45, 59, 59', 59") comprend les étapes suivantes :
(a) générer un plan virtuel orthogonal (37) par rapport à ladite direction d'incidence (55) ;
(b) positionner ledit plan virtuel à travers ladite région cible sur un bord distal de ladite région cible (53) ;
(c) déterminer une intersection entre ledit plan virtuel et ladite région cible (53), et
(d) agrandir ledit profil de faisceau d'ions par ladite intersection ;
(e) déplacer ledit plan virtuel en amont par rapport à ladite direction d'incidence (55), et
(f) répéter l'étape (c) à l'étape (e) au moins jusqu'à ce que ledit plan virtuel soit déplacé à travers ladite région cible.

4. Procédé selon l'une quelconque des revendications 1 à 3, comprenant en outre les étapes suivantes :
- faire varier ladite direction d'incidence dudit faisceau de particules et/ou de ladite région cible ;
- créer à nouveau et afficher à nouveau ledit profil de faisceau d'ions après chaque variation ;
- accepter respectivement parmi ladite direction d'incidence ayant varié et/ou ladite région cible ayant varié comme direction d'incidence finale et/ou région cible finale, et
- calculer une dose pour le faisceau d'ions sur la base de ladite direction d'incidence finale et/ou de ladite région cible finale.

5. Système de programmation de traitement, comprenant au moins un dispositif d'entrée (15), au moins un dispositif de sortie (17), et une unité de calcul (13), dans lequel ledit système de programmation de traitement (11) est apte à mettre en oeuvre un procédé selon l'une quelconque des revendications 1 à 4.

6. Support pouvant être lu par ordinateur présentant des instructions pouvant être exécutées par ordinateur pour mettre en oeuvre un procédé selon l'une quelconque des revendications 1 à 4.
